# EUROPÄISCHE PATENTSCHRIFT

(11) **EP 0 401 369 B1**
(45) Veröffentlichungstag und Bekanntmachung des Hinweises auf die Patenterteilung: **31.05.1995**
(21) Anmeldenummer: 89902412.9
(22) Anmeldetag: 27.01.1989
(51) Int. Cl.: C12P 21/00

(54) **VERFAHREN ZUR PRÄPARATIVEN GENEXPRESSION IN EINEM ZELLFREIEN SYSTEM DER KONJUGIERTEN TRANSKRIPTION/TRANSLATION**
METHOD FOR PREPARATIVE EXPRESSION OF GENES IN A CELL-FREE SYSTEM OF CONJUGATED TRANSCRIPTION/TRANSLATION
PROCEDE D'EXPRESSION PREPARATIVE DE GENES DANS UN SYSTEME EXEMPT DE CELLULES DE TRANSCRIPTION/TRANSLATION CONJUGUEES

(30) Priorität: 22.12.1988 SU 4618624
(43) Veröffentlichungstag der Anmeldung: 12.12.1990
(73) Patentinhaber: INSTITUT BELKA AKADEMII NAUK SSSR, Puschino 142292 (RU)
(72) Erfinder: BARANOV, Vladimir Ivanovich, Puschino, 142292 (RU); MOROZOV, Igor Jurievich, Puschino, 142292 (RU); SPIRIN, Alexandr Sergeevich, Moscow, 117420 (RU)
(74) Vertreter: von Füner, Alexander, Dr.
(86) Internationale Anmeldenummer: SU8900027
(87) Internationale Veröffentlichungsnummer: WO9007003

(56) Entgegenhaltungen:
- SCIENCE, Band 242, 25. November 1988, AAAS, Lancaster, PA (US); A.S. SPIRIN et al., Seiten 1162-1164#
- GENE, Band 6, Nr. 1, Mai 1979, Elsevier Press, Amsterdam (NL); J. COLLINS, Seiten 29-42#
- CHEMICAL ABSTRACT, Band 80, Nr. 23, 10. Juni 1974, Columbus, OH (US); G. ZUBAY, Seite 139, Nr. 129352x#
- B. KHEIMSA et al.(pad redaktsiei), "Transkriptsia i Translyatsia", Metody, 1987, Mir, Moskow (RU); Seiten 216-231#
- NUCLEIC ACIDS RESEARCH, Band 9, Nr. 18, 1981, IRL Press Ltd., London (GB); J.M. PRATT et al., Seiten 4459-4474#
- USPEKHI SOVREMENNOI BIOLOGII, Band 98, vyp. 3(6), 1984, Nauka, Moskow (RU); A.I. KOSIKOV et al., Seiten 338-352#

## Beschreibung

Die vorliegende Erfindung bezieht sich auf die Molekularbiologie und Biotechnologie, insbesondere betrifft sie Verfahren zur präparativen Genexpression in einem zellfreien System konjugierter Transkription/Translation. Bekanntlich sind Peptide oder Eiweiße Expressionsprodukte der Gene. Die genannten Stoffe werden umfassend in der Medizin als Bioregulatoren von biologischen Prozesse, als Heilmittel sowie als Diagnosesysteme eingesetzt. Beispielsweise, sind Peptid-Aktivatoren des Immunsystems, Peptid-Stimulatoren des Salzstoffwechsels, Eiweiße, die den Gehalt des Blutes an Zucker regulieren, und anderes mehr bekannt. Bekannt ist die Verwendung von Polypeptiden in der Landwirtschaft als Biostimulatoren, beispielweise Wachstumshormone.

Bekannt ist ein Verfahren zur präparativen Expression des genetischen Materials der Zelle nach der Methode der Gentechnik, das in dem Eindringen der fremden DNS, deren genetisches Material mit dem Apparat der Wirt-Zelle exprimiert wird, in die lebende Zelle beruht. Diese Methode findet breite Anwendung bei der großtechnischen Eiweißproduktion. Die Anwendungsmöglichkeiten dieses Verfahrens sind jedoch begrenzt, da die Isolierung der Expressionsprodukte des Gens, aufgrund der Letalität einiger Endprodukte für die Wirt-Zelle und der proteolytischen Degradation oder Aggregation des Exressionsproduktes eines fremden Gens, kompliziert ist. Der letzte Umstand ruft besondere Schwierigkeiten bei der Aussonderung der Polypeptide mit einer Länge von 15 bis 70 Aminosäureresten oder bei der Herstellung von Eiweißen mit künstlich abgeänderter Aminosäure-Sequenz hervor.

Aus dem Obendargelegten geht hervor, daß es die Methode der Gentechnik nicht möglich macht, die präparative Expression beliebiger Gene zu bewirken.

Es ist außerdem noch ein Verfahren zur Expression von Genen bekannt, das auf der Ausnutzung eines zellfreien Systems der konjugierten Transkription/Translation beruht.

Diese Methode beruht auf der Ausnutzung der Zellextrakte, in denen DNS-Molekeln verwendet werden. In solch einem System erfolgt die Vermehrung der mRNS nach den DNS-Molekeln und deren anschließende Translation. Die zellfreien Systeme der konjugierten Transkription/Translation sind zellspezifisch begrenzt und bewirken die Expression praktisch jedes beliebigen Gens in Form von erforderlichenfalls konstruierten DNS-Molekel.

Die Wirksamkeit solcher Verfahren ist sehr niedrig. Die Arbeitsdauer solch eines Systems beträgt zwischen 20 und 60 Minuten. Die Menge des synthetisierten Polypeptids übersteigt einen Wert von 3-10 pmol pro 1 ml Inkubationsgemisch nicht. Bekannt ist, beispielsweise, ein Verfahren zur Genexpression in einem zellfreien System der konjugierten Transkription/Translation ( Gene Vol. 6 1979, p.p. 29-42).

100»l zellfreies System der konjugierten Transkription/Translation enthält gemäß diesem bekannten Verfahren:
25 »l Extrakt S30, 5 »g DNS-Molekel, 19»g tRNS, 1,7 mM ATP, je 1 mM GTP, CTP, und UTP, 88 »M cAMP, 0,5 »g Folsäure, 37 mM Phosphoenolpyruvat, 2,6% Polyethylenglykol-6000, 88 »M [³⁵S]-Methionin, je 480 »M der übrigen 19 Aminosäuren in einem Puffer der folgenden Zusammensetzung:

77 mM Tris-Acetat, pH=8,2, 13 mM Magnesiumacetat, 13 mM Calciumacetat, 100 mM Kaliumacetat, 50 mM Ammoniumacetat, 2,4 mM DTT.

Man führt die Genexpression bei einer Temperatur von 37°C durch. Bei der Synthese werden in dem System Expressionsprodukte der Gene gespeichert, die ein Endprodukt in Form eines Polypeptids, Zerfallsprodukte in Form von AMP, ADP, GDP, CDP, UDP, Pyrophosphaten, anorganischen Phosphaten u.a. einschließen, die Inhibitoren des Systems der Genexpresion darstellen. Infolge der Inhibierung wird das System in 30-60 Minuten zum Stillstand gebracht. Die Menge des synthetisierten Polypeptids in 100»l solch eines Systems übersteigt 0,5-1 pmol nicht.

Die Hauptursachen der kurzen Arbeitsdauer des zellfreien Systems der konjugierten Transkription/Translation bestehen in folgendem:
1 . Das System enthält eine begrenzte Anzahl von Substraten. Die Herabsetzung ihrer Konzentration führt zur Inhibierung des jeweiligen Systems.
2. Die Zerfallsprodukte, die während der Arbeitsdauer des Systems in Form von AMP, ADP, GDP, UDP, Pyrophosphaten und Phosphaten entstehen, sind Inhibitoren des Systems.
3.Das Endprodukt kann auch das zellfreie System der konjugierten Transkription/Translation inhibieren.

Der Erfindung liegt die Aufgabe zugrunde, ein Verfahren zur Expression von Genen, die in Form von DNS-Molekeln vorliegen, in einem zellfreien System konjugierter Transkription/Translation zu entwickeln, das es ermöglicht, Polypeptide in präparativen Mengen durch Steigerung der Arbeitsdauer des Systems zu erhalten.

Diese Aufgabe wird dadurch gelöst, daß ein Verfahren zur präparativen Genexpression in einem zellfreien System konjugierter Transkription/Translation zur Verfügung gestellt wird, das als Substrate Aminosäuren, ATP, GTP, CTP und UTP enthält, unter Verwendung der Gene in Form von DNS-Molekeln unter Bildung der Genexpressionsprodukte, die Polypeptide, AMP, ADP, GDP, CDP, UDP, Pyrophosphat und anorganisches Phosphat umfassen, im System. Bei dem erfindungsgemäßen Verfahren werden während der Arbeit des zellfreien Systems der konjugierten Transkription/Translation, in dem Maße, in dem die Substrate unter Bildung der Produkte verbraucht werden, die Genexpressionsprodukte, die Polypeptide, AMP, ADP, GDP, CDP, Pyrophosphat und anorganisches Phosphat umfassen, aus dem System herausgeführt werden, gleichzeitig Substrate in Form von Aminosäuren, ATP, GTP, CTP, UTP zur Erhaltung ihrer Ausgangskonzentration in das System eingeführt.

Als Expressionssystem der Gene können in dem erfindungsgemäßen Verfahren sowohl prokaryotische als auch eukaryotische zellfreie Systeme der konjugierten Transkription/Translation verwendet werden. In dem erfindungsgemäßen Verfahren werden die Verhältnisse der Komponenten im Reaktionsgemisch, die Ionen- und Temperaturbedingungen der Syntheseführung angewendet, die für das gewählte System optimal sind. Der Bereich dieser Bedingungen ist breit und wird durch die Eigenschaften der Organismen, aus denen das zellfreie System hergestellt ist, ermittelt .

Das erfindungsgemäße Verfahren zur präparativen Genexpression ist frei von den Begrenzungen der Methoden der Gentechnik und kann für die Expression der Gene beliebiger Peptide und Eiweiße eingesetzt werden. Diese Eigenschaft ist für die Erzielung der biologisch aktiven Peptide und Eiweiße mit künstlich veränderter Aminosäuresequenz von großer Bedeutung.

Das erfindungsgemäße Verfahren gewährleistet die Genexpression im zellfreien System der konjugierten Transkription/Translation mit einer konstant hohen Geschwindigkeit innerhalb von 50 Stunden und darüber. Die Menge des Endproduktes beträgt dabei zwischen 20 und 400 »g pro 1 ml Reaktionsgemisch während einer Systemarbeitsdauer von 50 bis 100 Stunden. Dies ermöglicht eine großtechnische Produktion der Genexpressionsprodukte.

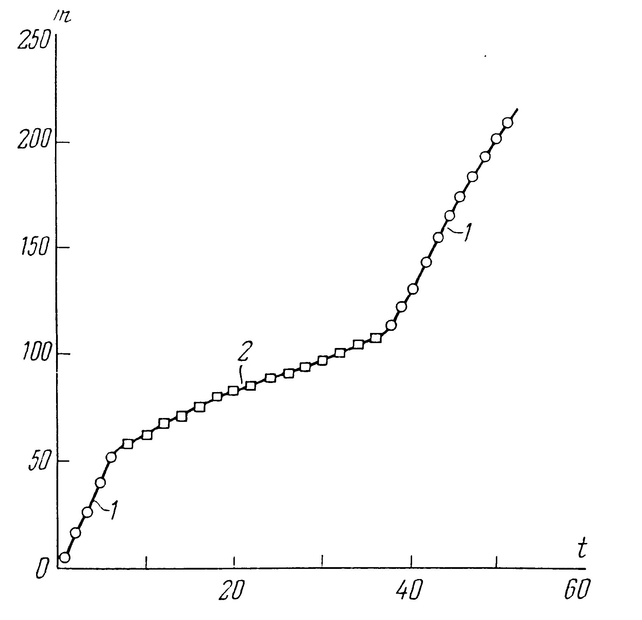

Nachstehend wird die Erfindung an Hand des Ausführungsbeispiels unter Bezugnahme auf die beigefügte Zeichnung erläutert, auf der die Abhängigkeit der Menge der synthetisierbaren Polypeptide von der Synthesezeit graphisch dargestellt ist.

Das Verfahren zur präparativen Genexpression im zellfreien System der konjugierten Transkription/Translation wird wie folgt durchgeführt.

Man stellt aus den Prokaryoten- oder Eukaryotenzellen einen Extrakt her, der alle Komponenten des Transkriptions- und Translationsapparates enthält, aber frei von endogener mRNS und DNS ist. Man fügt diesem Extrakt niedermolekulare Komponenten der Transkription und Translation (Aminosäuren, ATP, GTP, UTP, CTP) sowie ein Gen in Form einer DNS-Molekel hinzu. Man bringt das Reaktionsgemisch in einen Behälter ein, in dem der Prozeß der konjugierten Transkription/Translation abläuft. Um dem Prozeßstillstand vorzubeugen werden aus dem Behälter über eine semipermeable Membrane kontinuierlich Transkriptionsprodukte (anorganische Phosphate, ADP, GDP, CDP, UDP) und Translationsprodukte (synthetisiertes Polypeptid, AMP, GDP, anorganische Phosphate und Pyrophosphate) abgeführt. Gleichzeitig werden in den Behälter Aminosäuren, ATP, GTP, CTP und UTP gegeben, um deren Ausgangskonzentration zu erhalten. Das synthetisierte Polypeptid wird an der Säule mit einen Sorptionsmittel und die Transkriptions- und Translationsprodukte werden in einem Sonderbehälter für deren anschließende Regeneration gesammelt.

Zum besseren Verständnis der vorliegenden Erfindung wird folgendes konkretes Beispiel angeführt.

Präparative Genexpression des Plasmids pUD 18 in einem zellfreien System.

Man stellt ein zellfreies Standardsystem der konjugierten Transkription/Translation her. Das Plasmid pUD 18 enthält Gene der β-Laktamase und Dihydrofolatreduktase.
Lösung (A) enthält 55 mM Tris-Acetat (pH=8,2), 11 mM MgAc₂, 9,5 mM CaCl₂, 76 mM KAc, 1,5 mM DTT, 1,5% Polyethylenglykol-6000, 5 mM Phosphoenolpyruvat, 1,2 mM ATP, jeweils 0,8 mM GTP, CTP und UTP, 5 mg/ml Folsäure, 100 »M [³H]-Leucin mit einer spezifischen Radioaktivität von 230 mCi/mM und je 100 »M der übrigen Aminosäuren.

1 ml Reaktionsgemisch, hergestellt auf einem Puffer (A), enthält 430 »l Extrakt S30, 150 »g Plasmid pUD 18 und 200 »g tRNS.

Mit Hilfe des Standardsystems der konjugierten Transkription/Translation synthetisierte man während einer Inkubationszeit von 40 Minuten bei einer Temperatur von 37°C (Plateauwert) je 6 pmol β-Laktamase und Dihydrofolatreduktase pro 1 ml Inkubationgemisch.

In einem parallel verlaufenden Experiment auf einer kontinuierlich arbeitenden Anlage gibt man zu 1,0 ml Reaktionsgemisch eine Lösung (A) mit einer Geschwindigkeit von 3 ml/St oder 2 ml/St. Die Reaktionsprodukte werden aus dem Reaktionsgemisch mit derselben Geschwindigkeit über eine Ultrafiltrationsmembran XM-100 der Firma "Amikon", Holland, abgeführt. Die Kinetik der Genexpression der β-Laktamase und Dihydrofolatreduktase in diesem System ist in der Zeichnung durch ein Diagramm dargestellt, in dem auf der Abszissenachse die Dauer t der Synthese (in Stunden) und auf der Ordinatenachse die Menge m des anfallenden Produktes (in Mikrogramm) aufgetragen sind. Am Abschnitt 1 der kinetischen Kurve wird die Lösung (A) mit einer Geschwindigkeit von 3 ml/St und am Abschnitt 2 der kinetischen Kurve wird die Lösung (A) mit einer Geschwindigkeit von 2 ml/St zugeführt. Wie aus dem dargestellten Diagramm ersichtlich, hängt die Geschwindigkeit der Synthese von Polypeptiden von der Zuführungsgeschwindigkeit der Lösung (A) ab, nicht aber von der Arbeitsdauer des Systems ab.

Nach einer Arbeitsdauer dieses Systems von 50 Stunden synthetisiert man in 1 ml Reaktionsgemisch, je 4,1 nmol β-Laktamase und Dihydrofolatreduktase. Das entspricht 212 »g an Eiweißen der β-Laktamase und Dihydrofolatreduktase.

Auf diese Weise bewirkt das erfindungsgemäße Verfahren eine präparative Genexpression im zellfreien System, wobei die Synthese des Produktes um mehr als das 500fache gegenüber dem zellfreien Standardsystem der konjugierten Transkription/Translation gesteigert ist.

Das erfindungsgemäße Verfahren zur präparativen Genexpression im zellfreien System der konjugierten Transkription/Translation kann in der Biotechnologie zur Herstellung von Polypeptiden eingesetzt werden.

## Patentansprüche

1. Verfahren zur präparativen Genexpression in einem zellfreien System der konjugierten Transkription/Translation, auf der Basis eines Extraktes aus Pro- oder Eukaryotenzellen, der alle Komponenten des Transkriptions- und Translationsapparates enthält, aber frei von endogener mRNS und DNS ist, unter Verwendung der Gene in Form von DNS-Molekeln und unter Bildung der Genexpressionsprodukte, die Polypeptide, AMP, ADP, GDP, CDP, UDP, Pyrophosphat und anorganisches Phosphat umfassen, im System, dadurch **gekennzeichnet,** daß wärend der Arbeitsdauer des zellfreien Systems der konjugierten Transkription/Translation in dem Maße, in dem die Substrate unter Bildung der Produkte verbraucht werden, die Genexpressionsprodukte, die Polypeptide, AMP, ADP, GDP, CDP, UDP, Pyrophosphat und anorganisches Phosphat umfassen, aus dem System über eine Ultrafiltrationsmembrane abgeführt werden und gleichzeitig Substrate in Form von Aminosäuren, ATP, GTP, CTP, UTP zur Aufrechterhaltung ihrer Ausgangskonzentrationen dem System zugeführt werden.

## Claims

1. Process for preparative gene expression in a cell-free system of conjugated transcription/translation on the basis of an extract from prokaryote or eukaryote cells, which contains all the components of the transcription and translation apparatus but is free from endogenic mRNA and DNA, using the gene in the form of DNA molecules and forming the gene expression products, which comprise polypeptides, AMP, ADP, GDP, CDP, UDP, pyrophosphate and inorganic phosphate, in the system, characterised in that during the working duration of the cell-free system of conjugated transcription/translation to the extent that the substrates are used up in forming the products, the gene expression products, which comprise polypeptides, AMP, ADP, GDP, CDP, UDP, pyrophosphate and inorganic phosphate, are removed from the system by way of an ultra filtration membrane and at the same time substrates in the form of amino acids, ATP, GTP, CTP, UTP, are fed into the system in order to maintain their initial concentrations.

## Revendications

1. Procédé d'expression opératoire de gènes dans un système de translation/transcription conjuguée exempt de cellules, sur la base d'un extrait de cellules de pro- ou d'eucaryotes, qui contient tous les composants de l'appareil de transcription ou de translation, mais est exempt d'acide m-ribonucléique endogène et d'ADN, en utilisant les gènes sous la forme de molécules d'ADN et en formant des produits d'expression de gènes qui englobent peptides, AMP, ADP, GDP, CDP, UDP, pyrophosphate et phosphate inorganique, dans le système, ***caractérisé en*** **ce que**, pendant la durée de travail du système de translation/transcription conjuguée exempt de cellules, dans la même mesure selon laquelle les substrats sont consommés par formation des produits, les produits d'expression de gènes, qui englobent peptides, AMP, ADP, GDP, CDP, UDP, pyrophosphate et phosphate inorganique, sont évacués du système par l'intermédiaire d'une membrane d'ultra-filtration et, en même temps, des substrats sous la forme d'acides aminés, ATP, GTP, CTP, UTP, sont introduits dans le système pour maintenir leur concentration de départ.
